(19) Europäisches Patentamt
European Patent Office
Office européen des brevets

(11) **EP 3 284 432 B1**

(12) **EUROPEAN PATENT SPECIFICATION**

(45) Date of publication and mention
of the grant of the patent:
**17.03.2021 Bulletin 2021/11**

(51) Int Cl.:
***A61B 18/14*** *(2006.01)*  ***A61N 1/32*** *(2006.01)*
***A61B 90/98*** *(2016.01)*

(21) Application number: **17178725.2**

(22) Date of filing: **29.06.2017**

(54) **HANDLING AND CONTROL SYSTEM FOR EXPANDABLE ELECTRODES OF A HANDPIECE FOR USE IN AN ELECTRO-PORATION PROCESS**

HANDLING UND KONTROLLSYSTEM FÜR ERWEITERBAR ELEKTRODEN EINES HANDSTÜCKS ZUR VERWENDUNG IN EINEM ELEKTROPORATIONVERFAHREN

SYSTÈME DE MANIPULATION ET DE COMMANDE POUR ÉLECTRODES EXPANSIBLES D'UNE PIÈCE À MAIN DESTINÉE À ÊTRE UTILISÉE DANS UN PROCESSUS D'ÉLECTROPORATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.07.2016 IT 201600068691**

(43) Date of publication of application:
**21.02.2018 Bulletin 2018/08**

(73) Proprietor: **IGEA S.p.A.**
**41012 Carpi (IT)**

(72) Inventors:
• **CADOSSI, Ruggero**
**41012 CARPI (IT)**
• **MARAZZI, Donata**
**41012 CARPI (IT)**

• **BERTACCHINI, Claudio**
**41012 CARPI (IT)**
• **DE TERLIZZI, Francesca**
**31021 MOGLIANO VENETO (IT)**
• **RONCHETTI, Mattia**
**41012 CARPI (IT)**

(74) Representative: **Bongiovanni, Simone et al**
**Studio Torta S.p.A.**
**Via Viotti, 9**
**10121 Torino (IT)**

(56) References cited:
**US-A- 5 702 359       US-A1- 2012 150 172**
**US-A1- 2013 218 157   US-A1- 2013 296 995**
**US-A1- 2014 081 255**

**Description**

The present invention relates to a handling and control system for expandable electrodes of a handpiece for use in an electro-poration process.

As it is known, the objective of the electro-poration technique of a tissue is to homogeneously expose all the cells contained in the tissue to an electric field having an intensity exceeding a local threshold value in order to obtain a permeabilizing effect on the cell membranes. In some applications (for example ablation of tumour tissues) this local threshold value is in the order of 400V/cm (in general for values greater than 250V).

The tissue to be subjected to electro-poration must be penetrated homogeneously by the electric field, which must have a value above the threshold value in the whole of the volume of application of the electric field.

In order to make this electric field spatially effective, in many therapeutic applications use pairs of needle-shaped electrodes, which are inserted into the tissue to be treated at the same depth, maintaining the parallelism between the needles. For example, electrodes with fixed geometry, i.e., provided with pairs of electrodes rigidly fixed to each other, can be used in order to ensure parallelism between the needles.

The requirements of parallelism between the electrodes and penetration uniformity are not easy to achieve when wishing to treat tumour nodules located in places that are difficult to reach, such as hollow or visceral organs. This requirement is difficult to apply when wishing to avoid open surgery, for example in surgical procedures for hepatobiliary and pancreatic tumours.

To treat the aforesaid localized tumours, ablation techniques (e.g. radiofrequency ablation) have been proposed that use a handpiece provided with an expandable beam of electrodes shaped in the form of an elastic cable provided with needle-shaped end portions.

The needle-shaped end portions are inserted into the tumour nodule and the electrodes are subsequently positioned and/or expanded in the tissue. The electromagnetic energy issued by the electrodes produces strong heating of the surrounding tissue causing degenerative coagulation of this tissue.

An example of a handpiece provided with expandable electrodes of the aforesaid type is described in the patent EP-B-2.032.057, which illustrates a support assembly comprising an insulating body elongated along an axis and defining at its inside a plurality of inner channels each of which extends from a first distal end of the elongated body for a straight portion parallel to the axis, which is contiguous and communicating with a second curved portion that has radial distance with respect to the axis, increasing towards a second portion of the proximal end of the elongated body. Each curved portion leads to the second proximal end through a respective opening. The handpiece further comprises a plurality of flexible electrodes (needles) carried by the support assembly and mobile with respect to said body under the action of a pushing system of manual type; each electrode comprises an elastic cable made of conductive material covered with an insulating sheath and provided with a front uncovered portion that forms a needle-shaped active portion. Each elastic cable is housed inside a respective channel with the active portion that, when in use, protrudes from the respective opening. The manual pushing system acts on the rear portion of the flexible cables to produce a movement of these cables along an advancement direction in which the front portions of the cables that protrude from the support assembly advance along the axis and simultaneously are radially distanced from this axis.

The electrodes of the aforesaid type do not guarantee parallelism between the needle-shaped active portions. Moreover, the expandable electrodes do not provide for adjustment of the value of the electric field applied as a function of the positioning, which is fixed; therefore, it is not possible to proceed with successive steps of electro-poration for segmentation of the treatment of the nodule.

For those reasons, expandable electrodes of known type do not ensure electro-poration of all the cells of the tumour nodule.

If the parallelism between the electrodes is not maintained, the electric field applied in V/cm may differ greatly between the nearest and farthest tips of the electrodes. For example, in the nearest tips they could have values (V/cm) such as to determine a discharge through the tissue, or in the farthest tips not reach the threshold value V. The object of the present invention is to produce a handling and control system for expandable electrode which also allows the use of a handpiece provided with expandable electrodes in an electro-poration process, ensuring complete electro-poration of a volume of tissue.

Another example of an handpiece provided with expandable electrodes is described in US2012/0150172.

The aforesaid object is achieved by the present invention, as this relates to a system of the type described in claim 1.

The invention will now be illustrated with reference to the accompanying drawings that represent a preferred but non-limiting embodiment thereof, wherein:

- Fig. 1 illustrates, in a schematic longitudinal section, a handling and control system for expandable electrodes of a handpiece produced according to the dictates of the present invention;
- Fig. 2 illustrates handling and control operations of the expandable electrodes produced according to the present invention; and

- Fig. 3 schematizes the use of the expandable electrode;
- Fig. 4 illustrates a first variant of the system of Fig. 1; and

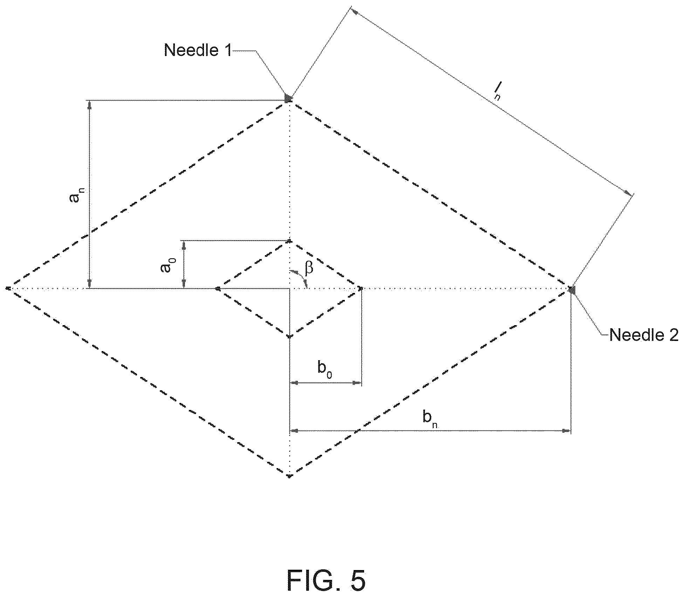

- Fig. 5 illustrates a second variant of the system of Fig. 1.

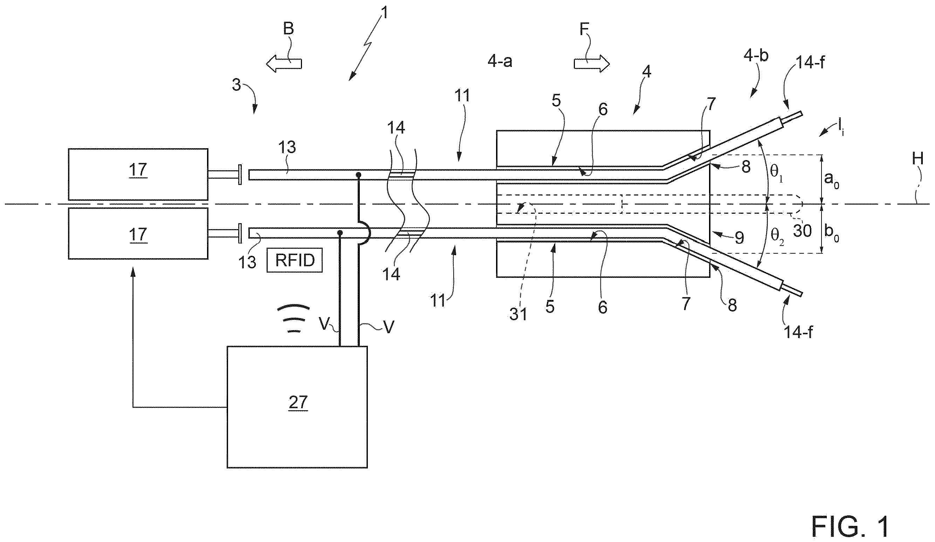

**Fig. 1 indicates with** 1, in its entirety, a handling and control system for handpiece 2 provided with expandable electrodes (illustrated schematically). The handpiece 2 can advantageously be used in an electro-poration process that does not form part of the subject-matter of this patent application, which only addresses the electrical, mechanical and control aspects of the system and the structure of the handpiece and of the electrodes.

The handpiece 2 comprises a support assembly - graspable electrodes 3 (which can be rigid or flexible - this graspable element is illustrated partially and schematically) provided with an elongated insulating body 4 along an axis H at one of its end (in the example a cylindrical body but the shape may vary) and defining at its inside a plurality of inner channels 5 (in the example channels with circular cross section) each of which extends from a first distal end 4-a of the elongated body 5 for a first straight portion 6 parallel to the axis H which is contiguous and communicating with a second straight portion 7 that extends along a direction which forms a divergence angle θ with respect to the axis H (and therefore also to the direction of extension of the first straight portion) and therefore has a radial distance with respect to the axis H increasing towards a second portion of the proximal end 4-b of the elongated body 4.

Typically, the divergence angle θ is variable from 5 to 45 degrees.

Each second straight portion 7 leads to the second proximal end 4-b through a respective opening 8 that opens in a wall 9 (in the example a flat wall perpendicular to the axis H but the shape could may vary) of this second portion of proximal end portion 4-b.

The handpiece 2 comprises a plurality of flexible electrodes 11 (to simplify the description, only one pair of electrodes is illustrated, but the number of electrodes can differ and form, for example, two, three, four or more pairs) carried by the support assembly 3 and by the elongated insulating body 4 and mobile with respect to the elongated insulating body 4 under the action of a pushing system 12 (illustrated schematically).

Each electrode 11 of the pair comprises a flexible cable 14 made of elastic conductive material (for example a steel wire) covered with an insulating sheath 13 and provided with a needle-shaped uncovered front portion 14-f (length comprised between 2 mm and 4 cm) that forms an active portion.

Each electrode 11 is housed inside a respective channel 5 with the active portion 14-f that, when in use, protrudes from the respective opening 8 positioned at the end of the channel 5. The pushing system 12 acts, for example, on the rear portion 14-b of the elastic cables 14 to perform a movement along an advancement direction F of these cables between a position at rest (not illustrated) in which the flexible electrodes are housed inside the respective channels 5 (and therefore the needle-shaped portions are not accessible ensuring safety) and a using position (Fig. 1) in which the front portions 14-f of the cables 14 and the electrode portions adjacent thereto, protrude from the support assembly 3.

The movement of the cables 14 along the advancement direction F produces the movement of the front portions 14-f along the axis H, axial distancing of these front portions 14-f from the second end 4-b and the radial distancing of the portions 14-f from the axis H due to the presence of the second rectilinear portions 7 that cause the advancement of the elastic cables along a direction inclined by the angle θ with respect to the axis H.

The portion of the elastic cables 14 that protrude from the openings 8 extends substantially rectilinearly due to the guide effect provided by the second straight portion 7. The protruding rectilinear portion thus forms an angle θ with respect to the axis H.

The movement of the cables 14 along a retraction direction B opposite the advancement direction F produces a movement of the front portions 14-f towards the second proximal end portion 4-b and radial movement of the portions 14-f towards the axis H. This movement continues until the portions 14-f return inside their respective channels 5 and the electrodes 11 are contained inside the support assembly 3. This position allows the electrodes to be kept safely, preventing accidental contact between the needle-shaped portions and an operator (not illustrated) handling the handpiece 2.

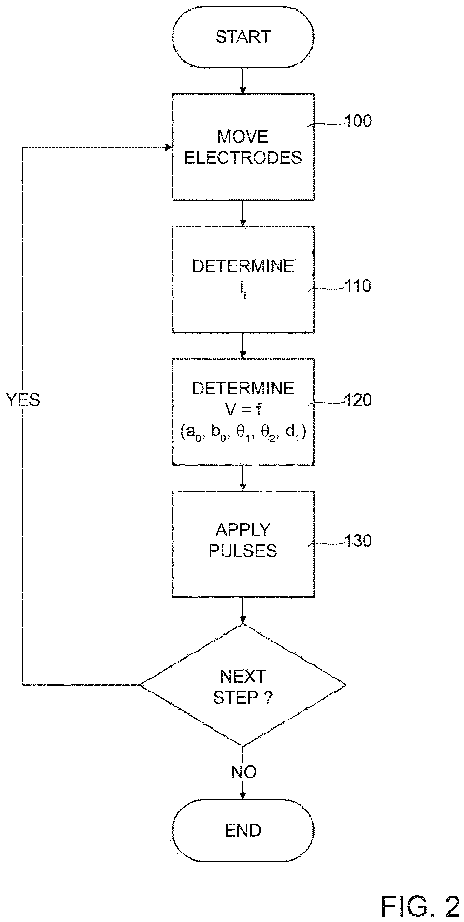

**According to** the present invention, there is provided an electronic control device 27 that controls the actuators 17 of the pushing system 12 and the voltage V applied to the electrodes 11 performing the operations described with reference to the flow-chart of Fig. 2.

The following operations are performed:

a) providing a command to the actuators 17 (in the example electromechanical actuators but one actuator could also be of a mechanical type) to perform an initial handling (block 100) of each cable 14 of the pair of electrodes 11 according to an initial pitch Δh performing an axial advancement of the front portion 14-f with respect to the second proximal end 4-b and a distancing of the front portion 14-f from the axis H;

b) determining for each electrode 11 of the pair the spacing or distance $l_i$, measured along a direction perpendicular to the axis H, between the tips of the front portions 14-f (block 110) of the pair of electrodes 11 (spacing between tips);

c) determining a voltage V (block 120) as a function of the spacing $l_i$ and applying to the electrodes 11 of the pair a pulsed signal (block 130) having maximum voltage V - the pulsed signal can have a different waveform (square, triangular, sinusoidal, sawtooth, etc.) and can have a fixed or variable frequency;

d) repeating the above steps a), b) e c) for a plurality k of steps k1, k2, ... ki, ...kn successive to the initial one and therefore for a plurality of increasing steps Δh1 Δh2 .. Δhi .. Δhn and increasing spacing values 11, 12 ... $l_i$ ...In between the electrodes of the pair so that the active portions 14-f of the electrodes 11 of the pair move in space in a three-dimensional application area; the voltage applied to the needle portions 14-f of the pair of electrodes 11 varies as a function of the spacing $l_i$ between the same electrodes increasing linearly with the increasing of the spacing.

Preferably the relation $V=f(l_i)$ between voltage V and $l_i$ is mapped in a table that outputs different increasing output values V for increasing values $l_i$, according to a linear law, for example those indicated in the table below:

| 11 =1 cm | V1 =1000V |
|---|---|
| 12 =2 cm | V2 =2000V |
| L3 =3 cm | V3 = 3000V |
| | |
| Li | Vi |
| | |
| | |
| | |
| Ln | Vn |

The n number k of steps can be set manually in order to define the maximum displacement of the active portions 14-f of the electrodes 11 with respect to the position taken by the active portions 14-f prior to the initial handling and consequently define a spacing $l_{MAX}$ between the tips of the active portions 14-f of the electrodes 11 of the pair. For example, the maximum spacing can take the value of 3 cm; a maximum voltage of 3000V is applied at this maximum spacing).

The spacing $l_i$ is determined based on the radial distance ao, bo between the outlet opening 8 of each second straight portion 7 of the pair of electrodes 11 and the axis H, according to the divergence angles $\theta 1$ e $\theta 2$ that each second straight portion 7 forms with respect to the axis H and according to the distance measured / estimated along the axis H between the wall 9 and the plane perpendicular to the axis H on which rest the tips of the end portions 14-f.

Typically, the following relation can be used:

$$l_i = a_0, + b_0 + d_i \tan (\theta 1) + d_i \tan (\theta 2).$$

where the distance di corresponds to the insertion depth of the electrodes measured along the axis H when the wall 9 is arranged in contact with a portion of human body and the electrodes 11 are inserted in this portion of human body.

The distance $d_i$ can be determined indirectly by means of a mapping that reports for each step a respective value di, namely

| k1 | d1 = 5 mm with (ao = $b_0$ = 2mm, $\theta 1 = \theta 2 = 45°$) |
|---|---|
| k2 | d2 = d1+5 mm |
| ... | |
| Ki | di = d(i-1)+5 mm |
| ... | |
| Kn | dn = d(n-1) + 5 mm |

Or can be determined directly by means of a sensor that measures the axial displacement of a rectilinear central electrode 30 (indicated with a dashed line) mobile along the axis H and housed in an axial cavity 31 of the elongated insulating body.

These relations are used by the block 110 to determine $d_i$.

In any case, the electronic control device 27 is configured to perform steps $\Delta h$ having a length smaller than the length of the front uncovered needle-shaped portion 14-f.

The maximum length of the active part can be selected as a function of the divergence angle $\theta°$, so that the length decreases as the angle increases and the electric field maintains a significant homogeneity in the volume affected by the electro-poration.

For example, according to the values contained in the attached table:

| Divergence angle: $\theta°$ | Active part mm |
| --- | --- |
| 5 | 18.0 |
| 10 | 9.1 |
| 15 | 6.2 |
| 22.5 | 4.3 |
| 45 | 3.0 |

the information relating to the values of ao, bo, at the divergence angles $\theta 1$ and $\theta 2$ and at the values taken by di for successive steps can be memorized - for each electrode or for each pair of electrodes - in the memory associated with an RFID device (Fig. 1) which is automatically activated when arranged in proximity of the electronic unit 27 to download the information ao, bo, $\theta 1$ and $\theta 2$ and di (if present in mapped form) and allow calculation of V. The RFID device could also provide other information, for example number of the pairs of electrodes 11 and length of the active portion 14-f.

Repetition of the steps a), b) e c) ensures that, for each position of the electrodes 11 in the three-dimensional application area, the potential V applied to each pair of electrodes, is updated so that the local value of the electric field is higher than the set threshold value thus obtaining the desired permeabilization effect on the cell membranes of the area of tissue corresponding to the three-dimensional application area.

**When in use,** the proximal end portion 4-b rests on or is placed in proximity to a portion of human body with the electrodes 11 arranged in the rest position. The electrodes 11 are then extracted and made to penetrate the tissues of the human body (naturally with the patient under local or total anaesthetic) until the needle-shaped portions 14-f reach a portion of tissue to be treated (for example a tumour nodule). During these positioning operations, no voltage is applied to the electrodes 11. The central electrode 30 - if present - also moves along the axis H and contributes to the measurement of the insertion depth $d_i$.

Subsequently, the electronic unit 27 takes control of the expandable electrode 2 and a voltage V is calculated/applied according to the values ao, bo, $\theta 1$ and $\theta 2$ (discharged by means of the use of RFID) and according to the value di measured or estimated.

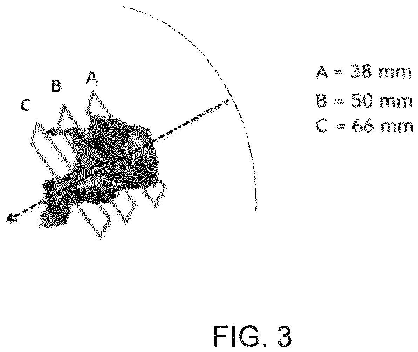

A signal having maximum voltage V is applied to each pair of electrodes 11 for a preset time interval, and electro-poration of a "slice" A of tumour nodule (see Fig. 3) is performed with an electric field that has a value locally above the threshold. As stated above, the voltage value applied is calculated so that it has a suitable value according to the spacing between the electrodes 11 and the thickness of which depends on the length of the active part of the needle 14-f (Fig. 3).

The actuator 17 applies to the electrodes 11 a first displacement $\Delta h$ performing an axial advancement $\Delta r$ of the front portion 14-f inside the nodule, the process to calculate the block 120 is repeated, and electro-poration of a second slice of nodule (slice "B", once again see Fig. 3) is performed with linearly increasing voltage. The spacing between the active portions 14-f of the electrodes is in fact increased and it is necessary to apply a higher voltage to ensure that the portion of the tissue arranged between the electrodes is penetrated by an electric field having a value above the threshold in each area of the nodule.

The actuator 17 applies to the electrodes 11 a second displacement $\Delta h$ performing a further axial advancement $\Delta r$ of the front portion 14-f inside and around the nodule, the calculation process of the block 120 is repeated, and electro-poration of a third slice of nodule (slice "C") is performed.

Repetition of the aforesaid operations allows electro-poration of N slices (for example ten slices, of the tumour nodule.

Following each "push" (i.e. displacement $\Delta h$) the electric field is "adjusted", namely recalculated as a function of the new positioning of the needle-shaped portions 14-f inside and around the tumour nodule. The process is repeated until the whole of the nodule (its depth) has been subjected to electro-poration.

If three electrodes 11 are provided housed inside the respective channels 5 that lead to the wall 9 through respective openings whose centres are arranged at the vertices of a triangle and form, one with respect to those adjacent thereto and with respect to the trace of the axis H, an angle $\beta$ (beta) measured on a plane perpendicular to the axis H, the electronic unit 27 determines the spacing $l_i$ between each pair of electrodes belonging to the group of three electrodes on the basis of:

$$l_i = \sqrt{a_n^2 + b_n^2 - 2a_n b_n cos\beta}$$

with

$$a_n = a_0, + d_n \tan (\theta 1)$$

$$b_n = b_0, + d_n \tan (\theta 2)$$

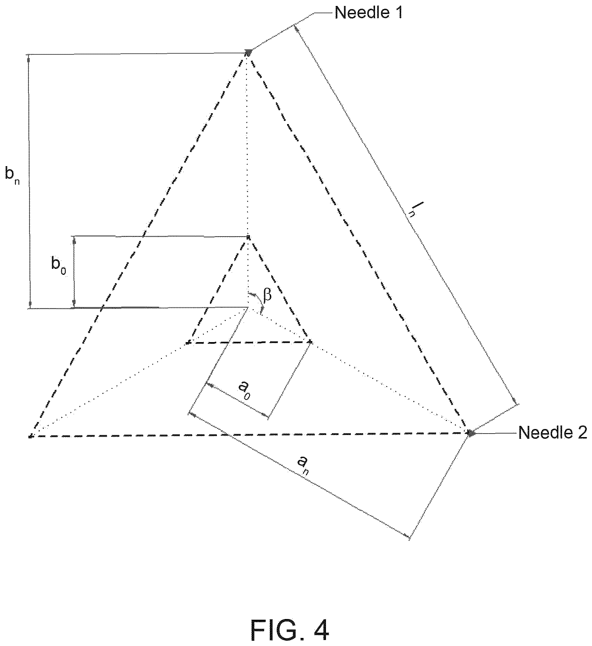

where a represents the radial distance between the centre of the opening 8 of a first electrode of the pair and the trace of the axis H, and b represents the radial distance between the centre of the opening 8 of a second electrode of the pair and the trace of the axis H. (Fig. 4).

If four electrodes 11 are provided housed inside respective channels 5 that lead onto the wall 9 through respective openings whose centres are arranged at the vertices of a rhombus and form, one with respect to those adjacent thereto and with respect to the trace of the axis H, an angle $\beta$ (beta) measured on a plane perpendicular to the axis H, the electronic unit 27 determines the spacing $l_i$ between each pair of electrodes belonging to the group of four electrodes on the basis of:

$$l_i = \sqrt{a_n^2 + b_n^2 - 2a_n b_n cos\beta}$$

with

$$a_n = a_0, + d_n \tan (\theta 1)$$

$$b_n = b_0, + d_n \tan (\theta 2)$$

where a represents the radial distance between the centre of the opening 8 of a first electrode of the pair and the trace of the axis H, and b represents the radial distance between the centre of the opening 8 of an adjacent second electrode of the pair and the trace of the axis H.

**Claims**

1. A handling and control system for expandable electrodes (2) of a handpiece for use in an electro-poration process comprising:

   a support assembly (3) provided with an elongated insulating body (4) along an axis H at one of its ends and defining in its inside a plurality of inner channels (5) each of which extends from a first distal end (4-a) of the elongated body (4) for a straight portion (6) parallel to the axis H which is contiguous and communicating with a second straight portion (7) that extends along a direction which forms a divergence angle with respect to the axis H having a radial distance with respect to the axis H increasing towards a second portion of the proximal end (4-b) of the elongated body; each second straight portion (7) leads to the second proximal end (4-b) through a respective opening (8);
   a plurality of flexible electrodes (11) carried by said support assembly and mobile with respect to said body under the action of a pushing system (12); each electrode comprising an elastic cable (14) made of conductive material covered with an insulating sheath (13) and provided with a front uncovered portion (14-f) that forms a needle-shaped active portion;
   each elastic cable (14) being housed inside a respective channel (5) with the active portion (14-f) that, when in use, protrudes from the respective opening (8);
the pushing system (12) acting on the flexible cables (14) to perform a movement of the cables themselves along an advancement direction (F) wherein the front portions of the cables (14) that protrude from the support assembly advance along said axis H and at the same time radially distance themselves from the axis H itself; the handling and control system (2) further comprising an electronic control device (27) configured to control the actuators (17) of the pushing system and the voltage applied to the electrodes to perform the following functions:

a) providing a command to the actuators (17) to perform an initial handling of each cable (14) according to an initial step Δh, performing an axial advancement of the front portion (14-f) with respect to the second proximal end (4-b) and a distancing of the front portion (14-f) from the axis H;
b) determining for each pair of electrodes (11) the spacing $l_i$, measured along a direction perpendicular to the axis (H), between the tips of the front portions (14-f) of the pair of electrodes (11) ;
c) determining a voltage V as a function of said spacing $l_i$, $V = f(l_i)$ and applying to each electrode a pulsed signal having maximum voltage equal to the determined voltage V;
d) repeating the above steps a), b) and c) for a plurality n of steps k successive to the initial one so that the active portions (14-f) of the electrodes move in space in a three-dimensional application area becoming distanced from each other; the voltage applied to the electrodes increases with the increasing of the spacing according to a set law and is such as to generate an electric field which ensures in said application area the complete electro-poration of the tissue.

2. *The system according to claim 1, wherein said electronic control device (27) is configured to determine said spacing $l_i$ based on the radial distance ao, bo between the outlet opening (8) of each second straight portion (7) of the pair of electrodes (11) and the axis (H), according to the divergence angles θ1 and θ2 that each second straight portion (7) form with respect to the axis H and according to the distance measured or estimated along the axis H between an end wall (9) of the second proximal end (4-b) and the plane perpendicular to the axis H on which rest the tips of the end portions (14-f).*

3. *The system according to claim 2, wherein said electronic control device (27) is configured with at least two electrodes for determining said spacing $l_i$ according to the following relationship:*

$$l_i = a_0, \ + b_0 +d_i \ \tan \ (\theta 1) \ + \ d_i \ \tan \ (\theta 2).$$

where the distance $d_i$ corresponds to the insertion depth of the electrodes measured along the axis H when the an end wall (9) of the elongated body is arranged in contact with a portion of human body when the electrodes (11) are inserted in this portion of human body.

4. *The system according to claim 2 or 3, wherein a RFID device is provided which is automatically activated when arranged in proximity to the electronic unit (27) to download to the electronic unit (27) the information associated with at least ao, bo and θ1 and θ2.*

5. *The system according to any one of the claims 2, 3 or 4, wherein sensors for directly detecting the value of $d_i$ are provided; where the distance $d_i$ corresponds to the insertion depth of the electrodes measured along the axis H when the an end wall (9) of the elongated body is arranged in contact with a portion of human body when the electrodes (11) are inserted in this portion of human body.*

6. *The system according to claim 5, wherein said sensors comprise a sensor that measures the axial displacement di of a rectilinear central electrode (30) mobile along the axis H and housed in a central axial cavity (31) of the elongated insulating body (4).*

7. *The system according to any one of the claims 2, 3 or 4, wherein said electronic control device (27) is configured to determine di indirectly by means of a mapping that reports, for each step k, a respective value of di.*

8. *The system according to any one of the preceding claims, wherein said electronic unit (27) is configured to set a number k of steps in order to define the maximum spacing between the active portions (14-f) of the electrodes (11).*

9. *The system according to any one of the preceding claims, wherein said electronic control device (27) is configured*

*to perform steps Δh having a length smaller than the length of the front uncovered needle-shaped portion (14-f).*

10. *The system according to any one of the preceding claims, wherein three electrodes (11) are provided, housed inside respective channels (5) leading to an end wall (9) of the second proximal end portion (4-b) through respective openings whose centres form, one with respect to those adjacent thereto and with respect to the trace of the axis H, an angle β (beta) measured on a plane perpendicular to the axis H;*
*the electronic unit (27) is configured to determine the spacing $l_i$ between each pair of electrodes belonging to the group of three electrodes on the basis of:*
*with*

$$l_i = \sqrt{a_n^2 + b_n^2 - 2a_n b_n \cos\beta}$$

*with*

$$a_n = a_0, + d_n \tan(\theta 1)$$

$$b_n = b_0, + d_n \tan(\theta 2)$$

*where a represents the radial distance between the centre of the opening (8) of a first electrode of the pair and the trace of the axis H, and b represents the radial distance between the centre of the opening (8) of a second electrode of the pair and the trace of the axis H.*

11. *The system according to any one of the claims from 1 to 9, wherein at least four electrodes are provided housed inside respective channels (5) which lead to an end wall (9) of the second portion of the proximal end (4-b) through respective openings whose centres are arranged at the vertices of a polygon and form, one with respect to those adjacent thereto and with respect to the trace of the axis H, an angle β (beta) measured on a plane perpendicular to the axis H,*
*the electronic unit (27) determines the spacing $l_i$ between each pair of electrodes belonging to the group of four or more electrodes on the basis of:*

$$l_i = \sqrt{a_n^2 + b_n^2 - 2a_n b_n \cos\beta}$$

*with*

$$a_n = a_0, + d_n \tan(\theta 1)$$

$$b_n = b_0, + d_n \tan(\theta 2)$$

*where a represents the radial distance between the centre of the opening 8 of a first electrode of the pair and the trace of the axis H, and b represents the radial distance between the centre of the opening 8 of an adjacent second electrode of the pair and the trace of the axis H.*

**Patentansprüche**

1. Handhabungs- und Steuersystem für erweiterbare Elektroden (2) eines Handstücks zur Verwendung in einem Elektroporationsvorgang, mit:

einer Stützanordnung (3), die an einem ihrer Enden entlang einer Achse H mit einem langgestreckten Isolierkörper (4) versehen ist und in ihrem Inneren mehrere innere Kanäle (5) bildet, die sich jeweils von einem ersten distalen Ende (4-a) des langgestreckten Körpers (4) über einen geraden Bereich (6) parallel zu der Achse H erstrecken, der an einen zweiten geraden Bereich (7) angrenzt und mit diesem verbunden ist, welcher sich in eine Richtung erstreckt, die einen Abweichungswinkel in Bezug auf die Achse H bildet, der eine radiale Entfernung in Bezug auf die Achse H aufweist, die in Richtung eines zweiten Bereichs des proximalen Endes (4-b) des langgestreckten Körpers zunimmt; jeder zweite gerade Bereich (7) führt durch eine jeweilige Öffnung (8) zu dem zweiten proximalen Ende (4-b);

mehreren flexiblen Elektroden (11), welche von der Stützanordnung getragen und in Bezug auf den Körper unter Einwirkung eines Schiebesystems (12) bewegbar sind; wobei jede Elektrode ein elastisches Kabel (14) aus leitfähigem Material aufweist, das mit einer isolierenden Hülle (13) bedeckt ist und mit einem vorderen unbedeckten Bereich (14-f) versehen ist, der einen nadelförmigen aktiven Bereich bildet;

wobei jedes elektrische Kabel (14) in einem jeweiligen Kanal (5) mit dem aktiven Bereich (14-f) angeordnet ist, welcher im Gebrauch aus dem jeweiligen Kanal (8) vorsteht;

wobei das Schiebesystem (12) auf die flexiblen Kabel (14) einwirkt, um eine Bewegung der Kabel selbst entlang einer Vorschubrichtung (F) durchzuführen, wobei die aus der Stützanordnung vorstehenden vorderen Bereiche der Kabel (14) sich entlang der Achse H vorbewegen und sich gleichzeitig radial von der Achse H selbst entfernen;

wobei das Handhabungs- und Steuersystem (2) ferner eine elektronische Steuervorrichtung (27) aufweist, die dazu ausgebildet ist, die Aktuatoren (17) des Schiebsystems und die an die Elektroden angelegte Spannung zur Durchführung der folgenden Funktionen zu steuern:

a) Ausgeben eines Befehls an die Aktuatoren (17) zur Durchführung einer anfänglichen Handhabung jedes Kabels (14) gemäß einem Anfangsschritt $\Delta h$, Durchführen eines axialen Vorschubs des vorderen Bereichs (14-f) in Bezug auf das zweite proximale Ende (4-b) und eines Beabstandens des vorderen Bereichs (14-f) von der Achse (H);

b) Bestimmen, für jedes Paar von Elektroden (11), des in einer zu der Achse (H) senkrechten Richtung gemessenen Abstands $l_i$ zwischen den Spitzen der vorderen Bereiche (14-f) der Paare von Elektroden (11);

c) Bestimmen einer Spannung V als eine Funktion des Abstands $l_i$, $V = f(l_i)$, und Anlegen eines gepulsten Signals an jede Elektrode, welches eine maximale Spannung aufweist, die gleich der bestimmten Spannung V ist;

d) Wiederholen der vorangehenden Schritte a), b) und c) für eine Vielzahl n von Schritten k nach dem Anfangsschritt, so dass die aktiven Bereiche (14-f) der Elektroden sich in einem dreidimensionalen Anwendungsbereich im Raum bewegen, wobei sie sich voneinander entfernen; die an die Elektroden angelegte Spannung nimmt mit der Zunahme des Abstands entsprechend einem festgelegten Gesetz zu und ist derart, dass sie ein elektrisches Feld erzeugt, das in dem Anwendungsbereich eine vollständige Elektroporation des Gewebes gewährleistet.

2.  System nach Anspruch 1, bei welchem die elektronische Steuervorrichtung (27) dazu ausgebildet ist, den Abstand $l_i$ basierend auf der radialen Entfernung $a_0$, $b_0$ zwischen der Ausgangsöffnung (8) jedes zweiten geraden Bereichs (7) des Paars von Elektroden (11) und der Achse (H) entsprechend den Abweichungswinkeln $\theta 1$ und $\theta 2$, welche jeder zweite gerade Bereich (7) in Bezug auf die Achse H bildet, und der entlang der Achse H gemessenen oder geschätzten Entfernung zwischen einer Stirnwand (9) des zweiten proximalen Endes (4-b) und der zu der Achse H senkrechten Ebene, in welcher die Spitzen der Endbereiche (14-f) liegen, zu bestimmen.

3.  System nach Anspruch 2, bei welchem die elektronische Steuervorrichtung (27) mit mindestens zwei Elektroden ausgebildet ist, um den Abstand $l_i$ entsprechend dem folgenden Verhältnis zu bestimmen:

$$L_i = a_0, + b_0 + d_i \tan(\theta 1) + d_i \tan(\theta 2),$$

wobei die Entfernung $d_i$ der Einführtiefe der Elektroden entspricht, welche, wenn die Stirnwand (9) des langgestreckten Körpers in Kontakt mit einem Bereich des menschlichen Körpers angeordnet ist, gemessen wird, wenn die Elektroden (11) in diesen Bereich des menschlichen Körpers eingeführt sind.

4.  System nach Anspruch 2 oder 3, bei welchem eine RFID-Vorrichtung vorgesehen ist, die, wenn sie in der Nähe der elektronischen Einheit (27) angeordnet ist, automatisch aktiviert wird, um die zumindest $a_0$, $b_0$ und $\theta 1$ und $\theta 2$ betreffenden Informationen in die elektronische Einheit herunterzuladen.

5.  System nach einem der Ansprüche 2, 3 oder 4, bei welchem Sensoren für das direkte Erkennen des Werts von $d_i$ vorgesehen sind; wobei die Entfernung $d_i$ der Einführtiefe der Elektroden entspricht, welche, wenn die Stirnwand (9) des langgestreckten Körpers in Kontakt mit einem Bereich des menschlichen Körpers angeordnet ist, gemessen wird, wenn die Elektroden (11) in diesen Bereich des menschlichen Körpers eingeführt sind.

**6.** System nach Anspruch 5, bei welchem die Sensoren einen Sensor aufweisen, der die axiale Verschiebung $d_i$ einer geradlinigen Mittelelektrode (30) misst, die entlang der Asche H bewegbar ist und in einem mittigen axialen Hohlraum (31) des langgestreckten Isolierkörpers (4) aufgenommen ist.

**7.** System nach einem der Ansprüche 2, 3 oder 4, bei welchem die elektronische Steuervorrichtung (27) dazu ausgebildet ist, $d_i$ indirekt mittels eine Mappings zu bestimmen, welche für jeden Schritt k einen jeweiligen Wert von $d_i$ angibt.

**8.** System nach einem der vorhergehenden Ansprüche, bei welchem die elektronische Einheit (27) dazu ausgebildet ist, eine Anzahl k von Schritten vorzugeben, um den maximalen Abstand zwischen den aktiven Bereichen (14-f) der Elektroden (11) zu definieren.

**9.** System nach einem der vorhergehenden Ansprüche, bei welchem die elektronische Einheit (27) dazu ausgebildet ist, Schritte $\Delta h$ durchzuführen, deren Länge geringer als die Länge des vorderen, unbedeckten nadelförmige Bereichs (14-f) ist.

**10.** System nach einem der vorhergehenden Ansprüche, bei welchem drei Elektroden (11) vorgesehen sind, die in jeweiligen Kanälen (5) aufgenommen sind, welche zu einer Stirnwand (9) des zweiten proximalen Endbereichs (4-b) durch jeweilige Öffnungen führen, deren Mitten jeweils in Bezug auf die benachbarten und in Bezug auf den Verlauf der Achse H einen auf einer zu der Achse H senkrechten Ebene gemessenen Winkel $\beta$ (beta) bilden; wobei die elektronische Einheit (27) dazu ausgebildet ist, den Abstand $l_i$ zwischen jedem Paar von Elektroden, die zu der Gruppe von drei Elektroden gehören, auf der Basis von

$$l_i = \sqrt{a_n^2 + b_n^2 - 2a_n b_n \cos\beta}$$

mit

$$a_n = a_0, + d_n \tan(\theta1)$$

$$b_n = b_0, + d_n \tan(\theta2)$$

zu bestimmen, wobei a die radiale Entfernung zwischen der Mitte der Öffnung (8) einer ersten Elektrode und dem Verlauf der Achse H angibt und b die radiale Entfernung zwischen der Mitte der Öffnung (8) einer zweiten Elektrode des Paars und dem Verlauf der Achse H angibt.

**11.** System nach einem der Ansprüche 1 bis 9, bei welchem mindestens vier Elektroden (11) vorgesehen sind, die in jeweiligen Kanälen (5) aufgenommen sind, welche zu einer Stirnwand (9) des zweiten proximalen Endbereichs (4-b) durch jeweilige Öffnungen führen, deren Mitten an den Scheitelpunkten eines Polygons angeordnet sind und in Bezug auf die benachbarten Öffnungen und in Bezug auf den Verlauf der Achse H einen auf einer zu der Achse H senkrechten Ebene gemessenen Winkel $\beta$ (beta) bilden; wobei die elektronische Einheit (27) den Abstand $l_i$ zwischen jedem Paar von Elektroden, die zu der Gruppe von vier oder mehr Elektroden gehören, auf der Basis von

$$l_i = \sqrt{a_n^2 + b_n^2 - 2a_n b_n \cos\beta}$$

mit

$$a_n = a_0, + d_n \tan(\theta1)$$

$$b_n = b_0, + d_n \tan(\theta2)$$

bestimmt, wobei a die radiale Entfernung zwischen der Mitte der Öffnung (8) einer ersten Elektrode und dem Verlauf der Achse H angibt und b die radiale Entfernung zwischen der Mitte der Öffnung (8) einer benachbarten zweiten Elektrode des Paars und dem Verlauf der Achse H angibt.

**Revendications**

1. Système de manipulation et de commande pour des électrodes expansibles (2) d'une pièce à main destinée à être utilisée dans un processus d'électroporation comprenant :

   un ensemble support (3) muni d'un corps isolant allongé (4) le long d'un axe H à l'une de ses extrémités, et définissant en son intérieur une pluralité de canaux internes (5) dont chacun s'étend depuis une première extrémité distale (4-a) du corps allongé (4) pour une partie droite (6) parallèle à l'axe H qui est contiguë et communiquant avec une seconde partie droite (7) qui s'étend le long d'une direction qui forme un angle de divergence par rapport à l'axe H présentant une distance radiale par rapport à l'axe H qui augmente vers une seconde partie de l'extrémité proximale (4-b) du corps allongé ; dans lequel chaque seconde partie droite (7) conduit à la seconde extrémité proximale (4-b) à travers une ouverture respective (8) ;
   une pluralité d'électrodes souples (11) portée par ledit ensemble support, et mobile par rapport audit corps sous l'action d'un système de poussée (12) ;
   chaque électrode comprenant un câble élastique (14) constitué d'un matériau conducteur recouvert d'une gaine isolante (13) et pourvu d'une partie avant non recouverte (14-f) qui forme une partie active en forme d'aiguille ;
   chaque câble élastique (14) étant logé à l'intérieur d'un canal respectif (5) avec la partie active (14-f) qui, lorsqu'elle est utilisée, fait saillie à partir de l'ouverture respective (8) ;
   le système de poussée (12) agissant sur les câbles élastiques (14) pour mettre en œuvre un déplacement des câbles eux-mêmes le long d'une direction d'avance (F) dans laquelle les parties avant des câbles (14), qui font saillie à partir de l'ensemble support, avancent le long dudit axe H et, simultanément, s'éloignent eux-mêmes radialement de l'axe H lui-même ;
   le système de manipulation et de contrôle (2) comprenant en outre un dispositif de contrôle électronique (27) configuré de manière à contrôler les actionneurs (17) du système de poussée et la tension appliquée aux électrodes, pour mettre en œuvre les fonctions suivantes consistant à :

   a) fournir une instruction, aux actionneurs (17), pour mettre en œuvre une manipulation initiale de chaque câble (14) selon une étape initiale $\Delta h$, mettre en œuvre une avance axiale de la partie avant (14-f) par rapport à la seconde extrémité proximale (4-b) et un éloignement de la partie avant (14-f) par rapport à l'axe H ;
   b) déterminer, pour chaque paire d'électrodes (11), l'espacement $l_i$, mesuré selon une direction perpendiculaire à l'axe (H), entre les pointes des parties avant (14-f) de la paire d'électrodes (11) ;
   c) déterminer une tension V en fonction dudit espacement $l_i$, $V = f(l_i)$, et appliquer, à chaque électrode, un signal pulsé présentant une tension maximale égale à la tension V déterminée ;
   d) répéter les étapes a), b) et c) ci-dessus pour une pluralité n d'étapes k successives à l'étape initiale, de sorte que les parties actives (14-f) des électrodes se déplacent dans l'espace, dans une zone d'application tridimensionnelle, en s'éloignant les unes des autres ; la tension appliquée aux électrodes augmente avec l'augmentation de l'espacement selon une loi établie, et est telle qu'elle génère un champ électrique qui garantit, dans ladite zone d'application, l'électroporation complète du tissu.

2. Système selon la revendication 1, dans lequel ledit dispositif de contrôle électronique (27) est configuré de manière à déterminer ledit espacement li sur la base de la distance radiale ao, bo entre l'ouverture de sortie (8) de chaque seconde partie droite (7) de la paire d'électrodes (11) et l'axe (H), selon les angles de divergence $\theta 1$ et $\theta 2$ que forme chaque seconde partie droite (7) par rapport à l'axe H, et selon la distance mesurée ou estimée le long de l'axe H entre une paroi d'extrémité (9) de la seconde extrémité proximale (4-b) et le plan perpendiculaire à l'axe H sur lequel reposent les pointes des parties d'extrémité (14-f).

3. Système selon la revendication 2, dans lequel ledit dispositif de contrôle électronique (27) est configuré avec au moins deux électrodes pour déterminer ledit espacement $l_i$ selon la relation donnée ci-dessous :

$$l_i = a_0, + b_0 + d_i \tan(\theta 1) + d_i \tan(\theta 2),$$

où la distance di correspond à la profondeur d'insertion des électrodes, mesurée le long de l'axe H lorsqu'une paroi d'extrémité (9) du corps allongé est agencée en contact avec une partie de corps humain, lorsque les électrodes (11) sont insérées dans cette partie de corps humain.

4. Système selon la revendication 2 ou 3, dans lequel est fourni un dispositif RFID qui est automatiquement activé lorsqu'il est agencé à proximité de l'unité électronique (27) pour télécharger, vers l'unité électronique (27), les informations associées à au moins ao, bo et les angles θ1 et θ2.

5. Système selon l'une quelconque des revendications 2, 3 ou 4, dans lequel des capteurs permettant de détecter directement la valeur de di sont fournis ; où la distance di correspond à la profondeur d'insertion des électrodes, mesurée le long de l'axe H, lorsque la paroi d'extrémité (9) du corps allongé est agencée en contact avec une partie de corps humain, lorsque les électrodes (11) sont insérées dans cette partie de corps humain.

6. Système selon la revendication 5, dans lequel lesdits capteurs comprennent un capteur qui mesure le déplacement axial di d'une électrode centrale rectiligne (30) mobile le long de l'axe H et logée dans une cavité axiale centrale (31) du corps isolant allongé (4).

7. Système selon l'une quelconque des revendications 2, 3 ou 4, dans lequel ledit dispositif de contrôle électronique (27) est configuré de manière à déterminer di indirectement, au moyen d'une mise en correspondance qui signale, pour chaque étape k, une valeur respective de di.

8. Système selon l'une quelconque des revendications précédentes, dans lequel ladite unité électronique (27) est configurée de manière à déterminer un nombre k d'étapes afin de définir l'espacement maximal entre les parties actives (14-f) des électrodes (11).

9. Système selon l'une quelconque des revendications précédentes, dans lequel ledit dispositif de contrôle électronique (27) est configuré de manière à mettre en œuvre des étapes Δh ayant une longueur inférieure à la longueur de la partie avant non recouverte en forme d'aiguille (14-f).

10. Système selon l'une quelconque des revendications précédentes, dans lequel trois électrodes (11) sont fournies, logées à l'intérieur de canaux respectifs (5) conduisant à une paroi d'extrémité (9) de la seconde partie d'extrémité proximale (4-b) à travers des ouvertures respectives dont les centres forment, l'un par rapport à ceux qui lui sont adjacents, et par rapport à la trace de l'axe H, un angle β (bêta) mesuré sur un plan perpendiculaire à l'axe H ; dans lequel l'unité électronique (27) est configurée de manière à déterminer l'espacement li entre chaque paire d'électrodes appartenant au groupe de trois électrodes, sur la base de :

$$l_i = \sqrt{a_n^2 + b_n^2 - 2a_n b_n \cos\beta}$$

avec

$$a_n = a_0, + d_n \tan(\theta1)$$

$$b_n = b_0, + d_n \tan(\theta2)$$

où « a » représente la distance radiale entre le centre de l'ouverture (8) d'une première électrode de la paire et la trace de l'axe H, et « b » représente la distance radiale entre le centre de l'ouverture (8) d'une seconde électrode de la paire et la trace de l'axe H.

11. Système selon l'une quelconque des revendications 1 à 9, dans lequel au moins quatre électrodes sont fournies, logées à l'intérieur de canaux respectifs (5) qui conduisent à une paroi d'extrémité (9) de la seconde partie de l'extrémité proximale (4-b), à travers des ouvertures respectives dont les centres sont agencés aux sommets d'un polygone et forment, l'un par rapport à ceux qui lui sont adjacents, et par rapport à la trace de l'axe H, un angle β (bêta) mesuré sur un plan perpendiculaire à l'axe H ; l'unité électronique (27) détermine l'espacement $l_i$ entre chaque paire d'électrodes appartenant au groupe de quatre électrodes ou plus, sur la base de :

$$l_i = \sqrt{a_n^2 + b_n^2 - 2a_n b_n \cos\beta}$$

avec

$$a_n = a_0, \; + d_n \tan(\theta 1)$$

$$b_n = b_0, \; + d_n \tan(\theta 2)$$

où « a » représente la distance radiale entre le centre de l'ouverture (8) d'une première électrode de la paire et la trace de l'axe H, et « b » représente la distance radiale entre le centre de l'ouverture (8) d'une seconde électrode adjacente de la paire et la trace de l'axe H.

$$l_i = \sqrt{a_n^2 + b_n^2 - 2a_n b_n \cos\beta}$$

FIG. 1

14

FIG. 2

A = 38 mm
B = 50 mm
C = 66 mm

FIG. 3

FIG. 4

FIG. 5

**REFERENCES CITED IN THE DESCRIPTION**

**Patent documents cited in the description**

- EP 2032057 B
- US 20120150172 A